# EUROPEAN PATENT APPLICATION

(11) **EP 3 088 081 A1**
(43) Date of publication of application: **02.11.2016**
(21) Application number: 14874115.0
(22) Date of filing: 24.12.2014
(51) Int. Cl.: B01J 31/06, B01J 27/053, C07C 29/10, C07C 31/18, C07C 67/08, C07C 69/14, C08G 8/28

(54) **RESIN SOLID ACID AND PRODUCTION METHOD THEREFOR**

(30) Priority: 26.12.2013 JP 2013269908; 19.12.2014 JP 2014257771
(71) Applicant: Futamura Kagaku Kabushiki Kaisha, Nagoya-shi, Aichi 450-0002 (JP)
(72) Inventor: KODAMA Atsushi, Kure-shi Hiroshima 737-0134 (JP); YAMADA Hirofumi, Kure-shi Hiroshima 737-0134 (JP); KAWAKAMI Ryuji, Kure-shi Hiroshima 737-0134 (JP); SHINGU Tatsuya, Kure-shi Hiroshima 737-0134 (JP)
(74) Representative: TBK
(86) International application number: PCT/JP2014/084067
(87) International publication number: WO 2015/098922

(57) **Abstract**

Provided is a resin solid acid capable of reducing intermediate stage production costs by omitting the synthetic resin carbonization step and allowing sulfo groups to be introduced directly into the synthetic resin, and a method for producing that resin solid acid. The resin solid acid is a sulfo group-modified resin obtained by introducing sulfo groups into a raw material resin in an uncarbonized state, the yield of the sulfo group-modified resin based on the weight of the uncarbonized raw material resin is 80% or more, the amount of sulfo groups in the sulfo group-modified resin is 1 mmol/g or more, and the raw material resin is in the form of a powder, granules or fibers. In addition, the method for producing the resin solid acid is a production method for obtaining a sulfo group-modified resin by comprising a step for adding a sulfonating agent in the form of any of sulfuric acid, fuming sulfuric acid or chlorosulfonic acid to a raw material resin in an uncarbonized state, and a step for heating the uncarbonized raw material resin at 200°C or lower.

## Description

### TECHNICAL FIELD

The present invention relates to a resin solid acid and a method for producing the same, and more particularly, to a resin solid acid, obtained by introducing sulfo groups (sulfonic acid groups) without carbonizing the raw material resin, and a method for producing the resin solid acid.

### BACKGROUND ART

Sulfuric acid is highly active, and is widely used as a catalyst when reacting hydrocarbon compounds. Examples of applications thereof include accelerating esterification reactions for obtaining higher fatty acid esters by reacting free higher fatty acids with alcohol, accelerating hydrolysis reactions of cellulose and other polysaccharides to monosaccharides, and accelerating alkylation reactions used to synthesize hydrocarbon fuels.

After having contributed to the acceleration of various types of reactions as a catalyst, sulfuric acid has been neutralized, washed and consumed each time it was used. Since sulfuric acid is a liquid, its recovery is not easy. As a result, it is currently common to dispose of sulfuric acid due to the difference in costs attributable to recovery treatment and the installation of new equipment for that purpose. However, in addition to having to neutralize and wash sulfuric acid, the burden becomes considerable when considering the additional need to treat the resulting wastewater so as to comply with environmental standards. Thus, there is a growing need for a catalyst, which in addition to being able to withstand continuous use as a catalyst, also offers a high level of user-friendliness in terms of being easier to isolate and recover after reacting.

Solid acids are an example of such a catalyst. Examples thereof include sulfuric acid-treated zirconia and fluorine resins introduced with sulfo (sulfonic acid) groups. The aforementioned zirconia has the shortcoming of low catalytic activity to the low concentration of sulfonic acid groups per unit weight. In addition, the aforementioned fluorine resins have the problem of being susceptible to heat, thereby limiting the reactive species to which they can be applied.

Therefore, carbon-based solid catalysts have been proposed for use as solid acids having both adequate catalytic activity and heat resistance (refer to, for example, Patent Document 1 and Patent Document 2). The solid acids disclosed in these patent documents are solid acids that have been put into the form of activated carbon or other carbon only by subjecting a hydrocarbon compound to carbonization treatment, followed by introducing sulfo groups. In this manner, the majority of carbon-based solid acids currently consist of solid acids that use activated carbon as the base material thereof.

As can be understood from the solid acids disclosed in patent documents and the like, calcination and carbonization of the resin or other hydrocarbon compound serving as the starting material was considered to be necessary. This is because an acid such as sulfuric acid or fuming sulfuric acid having potent oxidizing power is used when introducing sulfo groups. Consequently, the resin or other hydrocarbon compound ends up being decomposed due to reacting with sulfuric acid during sulfonation. Thus, in consideration of acid resistance and heat resistance, a carbide such as activated carbon can be said to be preferable for the base material introduced with sulfo groups.

However, in the case of carbonizing a resin and the like serving as the starting material under suitable conditions, calcination, carbonization, and activation equipment are required. Consequently, additional production costs corresponding to the costs for additional equipment are incurred in the stage prior to sulfonation. In addition, in the case of resins and other carbides, the number of sulfo groups introduced is known to decrease due to changes in functional groups present on the carbide surface depending on the calcination conditions. For this reason, previous studies on solid acid production have focused primarily on selection of the starting material and the control of carbonization and calcination conditions.

On the other hand, there are certain types of resins capable of serving as base materials of solid acids that have comparatively superior acid resistance and heat resistance. Extensive studies have been conducted on such resins to determine whether or not sulfo groups can be introduced directly without having to carry out carbonization. As a result, solid acids were able to be obtained that were observed to enable the introduction of a larger number of sulfo groups and demonstrate higher levels of catalytic activity than were conventionally thought.

### [Prior Art Documents]

### [Patent Documents]

[Patent Document 1] International Publication No. WO 2005/029508
[Patent Document 2] International Publication No. WO 2008/102913

### DISCLOSURE OF THE INVENTION

### [Problems to be Solved by the Invention]

With the foregoing in view, an object of the present invention is to provide a resin solid acid capable of reducing intermediate stage production costs by omitting the synthetic resin carbonization step and allowing sulfo groups to be introduced directly into the synthetic resin, and to provide a method for producing that resin solid acid.

### [Means for Solving the Problems]

Namely, the invention of claim 1 relates to a resin solid acid that is a sulfo group-modified resin, which uses for a raw material resin thereof a resin selected from among any of uncarbonized phenol resin, uncarbonized furan resin, uncarbonized urea resin, uncarbonized melamine resin and uncarbonized epoxy resin, and is obtained by introducing sulfo groups into the raw material resin.

The invention of claim 2 relates to the resin solid acid described in claim 1, wherein the yield of the sulfo group-modified resin based on the weight of the raw material resin is 80% or more.

The invention of claim 3 relates to the resin solid acid described in claim 1, wherein the amount of sulfo groups in the sulfo group-modified resin is 1 mmol/g or more.

The invention of claim 4 relates to the resin solid acid described in claim 1, wherein the raw material resin is an uncarbonized phenol resin or uncarbonized furan resin.

The invention of claim 5 relates to the resin solid acid described in claim 1, wherein the raw material resin is in the form of a powder, granules or fibers.

The invention of claim 6 relates to the resin solid acid described in claim 1, wherein the sulfo group-modified resin is used in a catalytic reaction.

The invention of claim 7 relates to the resin solid acid described in claim 1, wherein the sulfo group-modified resin is used in a hydrolysis reaction.

The invention of claim 8 relates to the resin solid acid described in claim 1, wherein the sulfo group-modified resin is used in an esterification reaction.

The invention of claim 9 relates to a method for producing the resin solid acid described in claim 1, in which a sulfo group-modified resin is obtained by comprising a step for using as a raw material resin a resin selected from any of uncarbonized phenol resin, uncarbonized furan resin, uncarbonized urea resin, uncarbonized melamine resin and uncarbonized epoxy resin, and adding a sulfonating agent to the raw material resin, and a step for the heating thereof.

The invention of claim 10 relates to the method for producing the resin solid acid described in claim 9, wherein the raw material resin is an uncarbonized phenol resin or uncarbonized furan resin.

The invention of claim 11 relates to the method for producing the resin solid acid described in claim 9, wherein the heating temperature in the heating step is 200°C or lower.

The invention of claim 12 relates to the method for producing the resin solid acid described in in claim 9, wherein the sulfonating agent is sulfuric acid, fuming sulfuric acid or chlorosulfonic acid.

### [Effects of the Invention]

According to the resin solid acid according to the invention of claim 1, since the resin solid acid is a sulfo group-modified resin obtained by using a resin selected from any of an uncarbonized phenol resin, uncarbonized furan resin, uncarbonized urea resin, uncarbonized melamine resin and uncarbonized epoxy resin as a raw material resin and introducing sulfo groups into the raw material resin, intermediate stage production costs can be reduced by omitting the carbonization step of the synthetic resin serving as the raw material and allowing sulfo groups to be introduced directly into the synthetic resin.

According to the resin solid acid according to the invention of claim 2, since the yield of the sulfo group-modified resin based on the weight of the raw material resin is 80% or more in the invention of claim 1, there is little decomposition of the synthetic resin caused by the sulfonating agent during the reaction for introducing sulfo groups, and production efficiency is extremely good.

According to the resin solid acid according to the invention of claim 3, since the amount of sulfo groups in the sulfo group-modified resin is 1 mmol/g or more in the invention of claim 1, favorable catalytic activity is demonstrated.

According to the resin solid acid according to the invention of claim 4, since the raw material resin is an uncarbonized phenol resin or uncarbonized furan resin in the invention of claim 1, resistance to sulfuric acid or other strong acids in the sulfonation stage is high and a resin solid acid can be stably obtained.

According to the resin solid acid according to the invention of claim 5, since the raw material resin is in the form of a powder, granules or fibers in the invention of claim 1, contact surface area with the reaction target of the resin solid acid increases, thereby allowing the obtaining of a favorable catalyst.

According to the resin solid acid according to the invention of claim 6, since the sulfo group-modified resin is used in a catalytic reaction in the invention of claim 1, the resin solid acid can be easily applied to a reaction device used in a catalytic reaction.

According to the resin solid acid according to the invention of claim 7, since the sulfo group-modified resin is used in a hydrolysis reaction in the invention of claim 1, the resin solid acid can be supplied both inexpensively and at a high level of activity to fields using large amounts of catalyst.

According to the resin solid acid according to the invention of claim 8, since the sulfo group-modified resin is used in an esterification reaction in the invention of claim 1, the resin solid acid can be supplied both inexpensively and at a high level of activity to fields using large amounts of catalyst.

According to the resin solid acid according to the invention of claim 9, since a sulfo group-modified resin is obtained in a method for producing the resin solid acid described in claim 1 by comprising a step for using as a raw material resin a resin selected from any of uncarbonized phenol resin, uncarbonized furan resin, uncarbonized urea resin, uncarbonized melamine resin and uncarbonized epoxy resin, and adding a sulfonating agent to the raw material resin, and a step for the heating thereof, a production method can be established that makes it possible to reduce intermediate stage production costs by omitting the carbonization step of the synthetic resin serving as the raw material and allowing sulfo groups to be introduced directly into the synthetic resin.

According to the resin solid acid according to the invention of claim 10, since the raw material resin is an uncarbonized phenol resin or uncarbonized furan resin in the invention of claim 9, resistance to sulfuric acid or other strong acids in the sulfonation stage is high and a resin solid acid can be stably obtained.

According to the resin solid acid according to the invention of claim 11, since the heating temperature in the heating step is 200°C or lower in the invention of claim 9, decomposition of the synthetic resin attributable to the potent oxidizing power of the sulfonating agent per se can be inhibited.

According to the resin solid acid according to the invention of claim 12, since the sulfonating agent is sulfuric acid, fuming sulfuric acid or chlorosulfonic acid in the invention of claim 9, it is a liquid that can be handled comparatively easily and procured inexpensively.

### BEST MODE FOR CARRYING OUT THE INVENTION

The following provides an explanation of the resin solid acid defined in the present invention along with the production method thereof. First, a synthetic resin is prepared that serves as the raw material of the resin solid acid. This synthetic resin is selected from among known resins, and more particularly, a resin is selected that has properties that make it resistant to decomposition during sulfonation to be subsequently described. More specifically, the synthetic resin is selected from among resins such as phenol resin, furan resin, urea resin, melamine resin or epoxy resin, engineering plastics or super engineering plastics. Among these, phenol resin or furan resin is suitable. As is disclosed in the examples to be subsequently described, these resins are used preferably since they are highly resistant to strong acid such as sulfuric acid present in the sulfonation stage. The use of these resins for the raw material resin results in greater constitutional homogeneity in comparison with conventional wood-based materials.

The aforementioned raw material resins are starting substances, and each of the resins is prepared in an uncarbonized state. A sulfonating agent is then added thereto. The raw material resins are then subjected to sulfonation while in that state. Sulfo groups are also referred to as sulfone groups or sulfonic acid groups, and are represented by -SO₂OH (or -SO₃H). The sulfo groups serve as active centers in a catalytic reaction since they are strongly acidic and electron attractive.

Increasing the contact surface area in the reaction and increasing the amount of sulfo groups introduced are considered with respect to the form of the raw material resin. Therefore, the raw material resin is preferably in the form of a powder, granules or fibers. Fibers include suitable forms in which resin fibers are processed into a woven fabric or nonwoven fabric. Since contact surface area with the reaction target of the resin solid acid also increases in the case the particle diameter or fiber diameter of the raw material resin is small, catalyst reactivity improves. In addition, powder, granules or fibers are also suitable for loading the resin solid acid into a reaction system, recovering the resin solid acid from the reaction system, and filling the resin solid acid into a reaction vessel and the like. There are no particular restrictions on the size of the raw material resin. However, if the cross-sectional diameter of the particle diameter or fiber diameter of individual powder particles or granules is excessively large, contact surface area with the reaction target of the resin solid acid decreases. Consequently, the particle diameter of a powder or granules is generally thought to preferably be 2 mm or less and fiber diameter (cross-sectional diameter) is generally thought to preferably be 1 mm or less.

An uncarbonized state refers to a state in which treatment, such as calcination or activation, has not been carried out that is related to carbonization of the raw material resin serving as a starting raw material. In the case of conventional solid acids, starting raw materials such as wood-based raw materials, petroleum pitch or synthetic resins were preliminarily subjected to treatment such as calcination, carbonization or activation to obtain carbides. Sulfo groups were then introduced therein. In contrast, in the case of the resin solid acid of the present invention, the conventional step for carbonizing raw materials in the production process has been completely omitted. In other words, a sulfonating agent is added to the raw material resin in the uncarbonized state (sulfonating agent addition step). Thus, the burden and costs associated with equipment and energy for obtaining carbides are reduced.

Next, it is difficult to introduce sulfo groups of the sulfonating agent into the raw material resin simply by mixing the raw material resin with the sulfonating agent. Therefore, the raw material resin and sulfonating agent are heated after mixing (heating step). This heating is of a degree that promotes a binding reaction between sulfo groups and the uncarbonized raw material resin, and is not carried out for the purpose of carbonizing the raw material resin. Namely, heating is carried out over a temperature range equal to or lower than the thermal decomposition temperature of the raw material resin of the starting raw material. In addition, there is also the risk of the raw material resin per se dissolving due to the potent oxidizing power of the sulfonating agent per se. Moreover, the presence of the sulfonating agent is also able to promote decomposition caused by heating even at a temperature equal to or lower than the aforementioned thermal decomposition temperature of the raw material resin per se.

Therefore, as is clear from the examples to be subsequently described, during heating, the heating temperature is within a temperature range of 200°C or lower, preferably 160°C or lower and more preferably 120°C or lower. Furthermore, although there are no particular restrictions on the lower limit of the heating temperature, from the viewpoint of promoting the sulfo group binding reaction, the lower limit temperature is room temperature or higher and preferably 40°C or higher. A suitable heating temperature in the heating step is selected in consideration of such factors as the type of raw material resin serving as a starting raw material or the degree of catalytic activity.

Sulfuric acid, fuming sulfuric acid or chlorosulfonic acid is used as the sulfonating agent used to introduce sulfo groups. These sulfonating agents are liquids, can be handled comparatively easily and can be procured inexpensively. The aforementioned fuming sulfuric acid consists of a mixture of sulfuric acid, disulfuric acid (pyrosulfuric acid) and sulfur trioxide in liquid and gaseous form, and all of these contribute to the manifestation of a dehydration condensation reaction to be subsequently described. Naturally, sulfonating agents other than those listed above can be used. The introduction of sulfo groups into the raw material resin is thought to be the result of sulfo groups bonding to hydroxyl groups or other functional groups of the raw material resin by dehydration condensation.

A sulfo group-modified resin can be obtained as a result of having introduced sulfo groups into the uncarbonized raw material resin. Subsequently, excess sulfonating agent is washed off by suitably rinsing with water after which excess moisture is removed. This completes production of the resin solid acid of the present invention. As is clear from this series of explanations, the sulfo group-modified resin in the resin solid acid of the present invention is not a resin that is introduced with sulfo groups during production in the raw material stage of the synthetic resin (during monomer synthesis) or during polymerization in the production stage of the synthetic resin. The sulfo group-modified resin is a sulfonated product obtained by newly introducing sulfo groups into the raw material resin at the stage the resin has already been completed as a resin (polymer). Thus, the sulfo group-modified resin is fundamentally different from resins such as sodium polystyrene sulfonate that initially have sulfo groups in molecules thereof.

When focusing on the main component of the resin solid acid in the form of the sulfo group-modified resin, in the relationship in which the weight of the resulting sulfo group-modified resin at the time of recovery is defined as (Wb) based on the initial weight (Wa) of the raw material resin serving as a starting raw material, the preferable yield (Y) is 80% or more. This yield (Y) refers to that represented by the equation "Y = (Wb/Wa) x 100". As can be understood from this equation, in the case of an ordinary reaction, the weight of the sulfo group-modified resin decreases from the weight at the start of the reaction accompanying loss or decomposition and the like that occurs during the course of the reaction. Here, maintaining a yield of 80% or more means that there is little decomposition of the raw material resin during the reaction for introducing sulfo groups. Thus, production efficiency is extremely good.

In addition, not only is decomposition of the raw material resin inhibited, but since the weight of the introduced sulfo groups per se is also added in, weight increases correspondingly. Thus, a yield of 100% or more is possible as a result of exceeding the amount lost during the course of the reaction.

The degree of catalytic activity of the resin solid acid of the present invention is influenced by the amount of sulfo groups in the sulfo group-modified resin. Therefore, from the viewpoint of being able to demonstrate favorable catalytic activity, the amount of sulfo groups in the sulfo group-modified resin is suitably 1 mmol/g or more, preferably 2 mmol/g or more and more preferably 4 mmol/g or more. Naturally, the greater the amount of sulfo groups, the higher the level of catalytic activity. However, due to restrictions on the amount of functional groups of the resin, the upper limit of the amount of sulfo groups is realistically 6 mmol/g to 7 mmol/g.

As will be understood from the examples to be subsequently described, the main application of the main component of the resin solid acid in the form of the sulfo group-modified resin is its use in a catalytic reaction. The catalytic reaction is attributable to sulfo groups present on the resin surface. The catalytic reaction is considered to be the same as conventional reactions that proceed in the presence of sulfuric acid. Thus, in the case of the resin solid acid, it can be easily applied to existing reaction devices used in catalytic reactions. In addition, since the sulfo group-modified resin is also inexpensive and demonstrates a high level of activity, it is attractive for use in industrial applications. There are countless types of catalytic reactions, and the sulfo group-modified resin can be applied to various types of catalytic reactions, including conventional reaction systems using sulfuric acid.

As examples of these catalytic reactions, the main component of the resin solid acid in the form of the sulfo group-modified resin is used in hydrolysis reactions and esterification reactions disclosed in the examples. In fields requiring these catalytic reactions, the market size is comparatively large and large amounts of catalysts are used. For these reasons, since the resin solid acid of the present invention can be supplied inexpensive and at a high level of activity, it can contribute to a reduction in production costs of the final product. Hydrolysis reactions are effective reactions when, for example, obtaining monosaccharides by decomposing polysaccharides such as cellulose. Esterification reactions are effective for producing, for example, pharmaceuticals and various types of chemical products.

As has been previously described, the first characteristic of the resin solid acid and method for producing that resin solid acid is that simplification of the production process can be realized. This constitutes a significant advance since carbonization of synthetic resins was initially thought to be essential. The next characteristic is that, despite omitting the carbonization step, there is no effect whatsoever on the desired level of catalytic activity. Moreover, uncarbonized synthetic resin demonstrates a higher level of catalytic activity (refer to the following examples).

### [Examples]

### (Raw Materials Used)

The following resins were used as synthetic resins (raw material resins) serving as starting raw materials of the resin solid acids of the examples.

Resins of the LPS® Series (Lygnite Inc. , particle diameter: 0.4 mm, 0.2 mm and 0.075 mm) were used as phenol resins (resole type). These resole-type phenol resins are in powdered and granular form.

Sulfo group-modified resins obtained by introducing sulfo groups into the aforementioned phenol resins (resole type) in which sulfo groups have been eliminated due to use are also treated as being able to be included in "raw material phenol resins" (resole type).

Kynol® KT-2800 (Gunei Chemical Industry Co., Ltd., fiber diameter: 14 µm) was used as phenol resin (novolac type). This novolac-type phenol resin is in the form of fibers.

The BEAPS® F series of crosslinked furfuryl alcohol resin particles (Asahi Organic Chemicals Industry Co., Ltd., particle diameter: 0.45 mm) was used as furan resin. This furan resin is in the form of granules.

Raw materials of the comparative examples and other raw materials were as indicated below.

Douglas fir sawdust was used as a wood-based raw material.

Sulfuric acid, fuming sulfuric acid and chlorosulfonic acid (all manufactured by Wako Pure Chemical Industry Co. , Ltd.) were used as sulfonating agents. The concentrations of fuming sulfuric acid shown in the column indicating the type of sulfonating agent in the tables (1%, 11.3%, 20% and 30%) were prepared using reagents of fuming sulfuric acid having different concentrations of 98% sulfuric acid and 30% fuming sulfuric acid.

### [Production of Resin Solid Acids Used in Examples]

### (Examples 1 to 16 and 20 to 25)

10 g of raw material resin serving as a starting raw material in the form of resole-type phenol resin or furan resin (powdered or granular) were weighed out and placed in a 500 mL three-neck flask followed by adding 100 mL aliquots of each of the aforementioned sulfonating agents. The reaction mixture was then stirred for 10 hours while maintaining the sulfonation temperatures shown in each table. Subsequently, the reaction mixture was washed with distilled water and repeatedly washed until the sulfuric acid ions present in the distilled water after washing were below the detection limit (Examples 1 to 16 and 20 to 25).

### (Examples 17 to 19)

3 g of novolac-type phenol resin (in the form of fibers) were weighed out and placed in a 500 ml three-neck flask followed by adding 200 mL to 300 mL of each of the aforementioned sulfonating agents. The reaction mixture was then stirred for 10 hours while maintaining the sulfonation temperatures shown in each table. Subsequently, the reaction mixture was washed with distilled water and repeatedly washed until the sulfuric acid ions present in the distilled water after washing were below the detection limit (Examples 17 to 19).

### (Example 26)

The sulfo group-modified resin of Example 1 was treated with hot water until the amount of sulfo groups reached 0.1 mmol/g or less. This treated resin was used as a phenol resin (resole type) serving as a starting raw material. 10 g of this treated resin (starting raw material) were weighed out and placed in a 500 mL three-neck flask followed by adding 100 mL of each of the aforementioned sulfonating agents and stirring for 10 hours while maintaining a sulfonation temperature of 80°C. Subsequently, the reaction mixture was washed with distilled water and repeatedly washed until the sulfuric acid ions present in the distilled water after washing were below the detection limit (Example 26).

### (Production of Solid Acids Used in Comparative Examples)

### (Comparative Example 1)

Comparative Example 1 is an example of sulfonating an uncarbonized wood-based raw material. 10 g of wood-based raw material were weighed out and placed in a 500 mL three-neck flask followed by adding 100 mL of one of the aforementioned sulfonating agents in the form of fuming sulfuric acid. However, the wood-based raw material ended up decomposing during heating at 80°C and was unable to be recovered.

### (Comparative Examples 2 to 4)

Comparative Examples 2 to 4 are examples of sulfonating a carbonized wood-based raw material. The wood-based raw material was initially arranged on a metal plate, the temperature was raised to the heating temperature corresponding to each comparative example in the tables (300°C to 400°C) while maintaining an inert atmosphere with nitrogen gas using a muffle furnace (trade name: INH-51N1, Koyo Thermo Systems Co., Ltd.), and that temperature was then maintained for 1 hour. Following completion of heating and cooling, the wood-based raw material was removed from the muffle furnace to obtain carbonized products of the wood-based material of each comparative example.

10 g aliquots of each carbonized product of the wood-based raw materials of each comparative example were weighed out and placed in a 500 mL three-neck flask followed by adding 100 mL of fuming sulfuric acid. The reaction mixture was stirred for 10 hours while maintaining the reaction temperature at 80°C. Subsequently, the wood-based raw material was washed with distilled water and repeatedly washed until the sulfuric acid ions present in the distilled water after washing were below the detection limit (Comparative Examples 3 and 4). Furthermore, the wood-based raw material of Comparative Example 2 was unable to be recovered since it decomposed during sulfonation.

### (Comparative Examples 5 to 11)

The resole-type phenol resin, novolac-type phenol resin and furan resin used in the examples were heated to the heating temperature corresponding to each comparative example in the tables (300°C to 600°C) while maintaining an inert atmosphere with nitrogen gas using the aforementioned muffle furnace, and that temperature was then maintained for 1 hour. Following completion of heating and cooling, the synthetic resins were removed from the muffle furnace to obtain carbonized products of the synthetic resin of each comparative example.

10 g aliquots of each carbonized product of the synthetic resins of Comparative Examples 5 to 9 (resole-type phenol resins) were weighed out and placed in a 500 mL three-neck flask followed by adding 100 mL of fuming sulfuric acid. The reaction mixture was stirred for 10 hours while maintaining the reaction temperature at 80°C. Subsequently, the synthetic resins were washed with distilled water and repeatedly washed until the sulfuric acid ions present in the distilled water after washing were below the detection limit (Comparative Examples 5 to 9).

3 g of the carbonized product of the synthetic resin of Comparative Example 10 (novolac-type phenol resin) were weighed out and placed in a 500 mL three-neck flask followed by adding 300 mL of fuming sulfuric acid. The reaction mixture was stirred for 10 hours while maintaining the reaction temperature at 80°C. Subsequently, the synthetic resin was washed with distilled water and repeatedly washed until the sulfuric acid ions present in the distilled water after washing were below the detection limit (Comparative Example 10).

10 g of the carbonized product of the synthetic resin of Comparative Example 11 (furan resin) were weighed out and placed in a 500 mL three-neck flask followed by adding 100 mL of fuming sulfuric acid. The reaction mixture was stirred for 10 hours while maintaining the reaction temperature at 80°C. Subsequently, the synthetic resin was washed with distilled water and repeatedly washed until the sulfuric acid ions present in the distilled water after washing were below the detection limit (Comparative Example 11).

### (Measurement of Physical Properties and Activity)

### (Yield)

Yield (Y₁) of the uncarbonized raw materials of the examples was determined by recovering the sulfo group-modified resin following sulfonation and weighing the amount thereof (Wb) based on the initial weight (Wa) of the synthetic resin prior to the sulfonation stage, and determining the quotient thereof. Namely, yield in the examples refers to that determined using the equation "Y₁(%) = (Wb/Wa) x 100". In order to ensure accurate measurement, nearly the entire amount of washed sulfo group-modified resin was recovered using a filtration membrane made of fluorine resin (JCWP04700, Omnipore Membrane, pore diameter: 10 µm) followed by drying at 100°C over the course of 10 hours. The weight of this dried product was taken to be the weight of the sulfo group-modified resin.

Yield (Y₂) of the carbonized raw materials of the comparative examples was based on the initial weight (Wa) of the wood-based raw material or synthetic resin serving as the starting raw material. The carbonized product was then recovered following carbonization and the weight thereof (Wp) was measured. Yield (Y_{c}) of the raw material at the carbonization stage was then determined. More specifically, yield (Y_{c}) was determined using the equation "Y_{c} = (Wp/Wa)".

Continuing, sulfonated products were recovered following sulfonation followed by measuring the weight thereof (Wq) based on the weight of the carbonized product (Wp). Yield (Yₛ) in the sulfonation stage was then determined. More specifically, yield (Yₛ) was determined using the equation "Yₛ = (Wq/Wp)". In this case as well, nearly the entire amount of the washed sulfonated product was recovered using the aforementioned filtration membrane made of fluorine resin followed by drying at 100°C over the course of 10 hours. The weight of this dried product was taken to be the weight of the sulfonated product.

The final yield (Y₂) of the carbonized raw materials of the comparative examples is the product of the yields determined at intermediate stages. Therefore, yield (Y₂) was determined using the equation "Y₂(%) = (Yc x Ys) x 100".

### (Measurement of Sulfur Content and Amount of Sulfo Groups)

Solid acids of the examples and comparative examples were heated to 100°C and dried during elemental analysis. The compositions of elements contained in each of the solid acids were analyzed at a combustion temperature of 1000°C using the ICS-1000 automated combustion ion chromatograph manufactured by Dionex Corp., the AQF-1000 combustion system manufactured by Mitsubishi Chemical Analytech Co., Ltd., the GA-100 absorption system manufactured by Mitsubishi Chemical Analytech Co., Ltd., and the WS-100 pump unit manufactured by Mitsubishi Chemical Analytech Co., Ltd. The resulting sulfur content (mmol/g) (converted to wt%) was taken to be equal to the amount of sulfo groups, and was determined as the amount of sulfo groups in the solid acid per unit weight (mmol/g).

### (Measurement of Catalytic Activity)

### (Measurement of Hydrolysis Reaction)

The solid acids of the examples and comparative examples were heated to 100°C and dried. 0.1 g of solid acid was placed in a sample bottle followed by the addition of 0.12 g of cellobiose and 0.7 mL of water and reacting for 60 minutes while maintaining the temperature at 90°C. Following cooling after the reaction, 2.3 mL of water were added followed by filtering with a syringe filter. The filtrate was filled into a high-performance liquid chromatograph (HPLC, Model RID-10A, Shimadzu Corp.) using the Aminex HPX-87H column manufactured by Bio-Rad Laboratories, K.K.), and the amounts of sugars formed by hydrolysis of the cellobiose were determined from the peak area ratios of monosaccharides such as glucose. This was then converted to the amount of cellobiose (µmol) that was decomposed by reacting for 1 hour per 1 g of solid acid (µmol ·g⁻¹ ·h⁻¹).

### (Measurement of Esterification Reaction)

The solid acids of the examples and comparative examples were heated to 100°C and dried. 0.2 g of solid acid were transferred to a flask and vacuum-dried (0.4 Pa or lower) for 1 hour at 150°C. Following completion of vacuum drying, 58.5 mL (1.0 mol) of ethanol and 5.742 mL (0.1 mol) of acetic acid were added to the solid acid followed by reacting for 60 minutes while maintaining the temperature at 70°C. Following cooling after the reaction, the reaction mixture was filtered with a syringe filter. The formed amount of ethyl acetate contained in the filtrate was determined by gas chromatography using the GC-2014 FID gas chromatograph manufactured by Shimadzu Corp., and the Agilent J&W GC column and DB-WAX capillary column manufactured by Agilent Technologies Inc. This was then converted to the amount decomposed by reacting for 1 minute per 1 g of solid acid (mmol ·g⁻¹ ·min⁻¹).

The results obtained for the solid acids of the examples and comparative examples produced and measured as described above are shown in Tables 1 to 8. Raw materials (type, particle size (particle diameter), carbonization conditions (temperature), sulfonating agent (type, concentration), sulfonation conditions (temperature, yield, sulfur content) and catalyst evaluation (hydrolysis reaction rate, esterification reaction rate) are shown in each of the tables in that order.

**[Table 1]**

| | Sample No. | Ex.1 | Ex. 2 | Ex. 3 | Ex.4 | Ex. 5 |
|---|---|---|---|---|---|---|
| Raw materials | Wood-based raw material | | | | | |
| | Phenol resin (resole-type) | ○ | ○ | ○ | ○ | ○ |
| | Phenol resin (novolac-type) | | | | | |
| | Furan resin | | | | | |
| | Powder or granules: particle diameter (mm) | 0.2 | 0.2 | 0.2 | 0.2 | 0.2 |
| | Fibers: fiber diameter (µm) | | | | | |
| Carbonization conditions | Carbonization temperature (°C) | Uncarbonized | Uncarbonized | Uncarbonized | Uncarbonized | Uncarbonized |
| Sulfonating agent | Type | Fuming sulfuric acid | Fuming sulfuric acid | Fuming sulfuric acid | Fuming sulfuric acid | Fuming sulfuric acid |
| | Concentration (%) | 11.3 | 11.3 | 11.3 | 11.3 | 11.3 |
| Sulfonation conditions | Sulfonation temperature (°C) | 80 | 40 | 120 | 160 | 200 |
| | Yield (%) | 154 | 126 | 161 | 122 | 85 |
| | Sulfur content (wt%) | 16.1 | 6.8 | 15.9 | 12.7 | 12.8 |
| | Sulfo groups (mmol/g) | 5.0 | 2.1 | 5.0 | 4.0 | 4.0 |
| Catalyst evaluation | Hydrolysis reaction rate (µmol•g⁻¹•h⁻¹) | 1265 | 456 | 1447 | 1353 | 1257 |
| | Esterification reaction rate (mmol·g⁻¹·min⁻¹) | 3.0 | 1.4 | 2.8 | 3.1 | 2.3 |

**[Table 2]**

| | Sample No. | Ex. 6 | Ex. 7 | Ex. 8 | Ex. 9 | Ex. 10 |
|---|---|---|---|---|---|---|
| Raw materials | Wood-based raw material | | | | | |
| | Phenol resin (resole-type) | ○ | ○ | ○ | ○ | ○ |
| | Phenol resin (novolac-type) | | | | | |
| | Furan resin | | | | | |
| | Powder or granules: particle diameter (mm) | 0.2 | 0.2 | 0.2 | 0.075 | 0.4 |
| | Fibers: fiber diameter (µm) | | | | | |
| Carbonization conditions | Carbonization temperature (°C) | Uncarbonized | Uncarbonized | Uncarbonized | Uncarbonized | Uncarbonized |
| Sulfonating agent | Type | Fuming sulfuric acid | Fuming sulfuric acid | Fuming sulfuric acid | Fuming sulfuric acid | Fuming sulfuric acid |
| | Concentration (%) | 1.0 | 20.0 | 30.0 | 11.3 | 11.3 |
| Sulfonation conditions | Sulfonation temperature (°C) | 80 | 80 | 80 | 80 | 80 |
| | Yield (%) | 154 | 121 | 108 | 154 | 170 |
| | Sulfur content (wt%) | 15.0 | 9.0 | 10.1 | 15.0 | 16.3 |
| | Sulfo groups (mmol/g) | 4.7 | 2.8 | 3.2 | 4.7 | 5.1 |
| Catalyst evaluation | Hydrolysis reaction rate (µmol•g⁻¹•h⁻¹) | 1661 | 520 | 516 | 962 | 1047 |
| | Esterification reaction rate (mmol·g⁻¹·min⁻¹) | 3.8 | 1.6 | 1.5 | 2.7 | 3.1 |

**[Table 3]**

| | Sample No. | Ex. 11 | Ex. 12 | Ex. 13 | Ex. 14 | Ex. 15 |
|---|---|---|---|---|---|---|
| Raw materials | Wood-based raw material | | | | | |
| | Phenol resin (resole-type) | ○ | ○ | ○ | ○ | ○ |
| | Phenol resin (novolac-type) | | | | | |
| | Furan resin | | | | | |
| | Powder or granules: particle diameter (mm) | 0.2 | 0.2 | 0.2 | 0.2 | 0.2 |
| | Fibers: fiber diameter (µm) | | | | | |
| Carbonization conditions | Carbonization temperature (°C) | Uncarbonized | Uncarbonized | Uncarbonized | Uncarbonized | Uncarbonized |
| Sulfonating agent | Type | Sulfuric acid | Sulfuric acid | Sulfuric acid | Sulfuric acid | Sulfuric acid |
| | Concentration (%) | - | - | - | - | - |
| Sulfonation conditions | Sulfonation temperature (°C) | 40 | 80 | 120 | 160 | 200 |
| | Yield (%) | 140 | 151 | 133 | 132 | 124 |
| | Sulfur content (wt%) | 13.3 | 14.9 | 14.2 | 12.4 | 10.0 |
| | Sulfo groups (mmol/g) | 4.1 | 4.6 | 4.4 | 3.9 | 3.1 |
| Catalyst evaluation | Hydrolysis reaction rate (µmol•g⁻¹•h⁻¹ ) | 1071 | 1627 | 1504 | 1354 | 886 |
| | Esterification reaction rate (mmol·g⁻¹·min⁻¹) | 2.8 | 3.4 | 3.2 | 2.2 | 2.8 |

**[Table 4]**

| | Sample No. | Ex. 16 | Ex. 17 | Ex. 18 | Ex. 19 | Ex. 20 |
|---|---|---|---|---|---|---|
| Raw materials | Wood-based raw material | | | | | |
| | Phenol resin (resole-type) | ○ | | | | |
| | Phenol resin (novolac-type) | | ○ | ○ | ○ | |
| | Furan resin | | | | | ○ |
| | Powder or granules: particle diameter (mm) | 0.2 | | | | 0.45 |
| | Fibers: fiber diameter (µm) | | 14 | 14 | 14 | |
| Carbonization conditions | Carbonization temperature (°C) | Uncarbonized | Uncarbonized | Uncarbonized | Uncarbonized | Uncarbonized |
| Sulfonating agent | Type | Chlorosulfonic acid | Fuming sulfuric acid | Fuming sulfuric acid | Fuming sulfuric acid | Fuming sulfuric acid |
| | Concentration (%) | - | 11.3 | 11.3 | 11.3 | 11.3 |
| Sulfonation conditions | Sulfonation temperature (°C) | 80 | 80 | 120 | 160 | 80 |
| | Yield (%) | 137 | 118 | 168 | 155 | 137 |
| | Sulfur content (wt%) | 14.2 | 6.5 | 11.1 | 9.9 | 9.0 |
| | Sulfo groups (mmol/g) | 4.4 | 2.0 | 3.5 | 3.1 | 2.8 |
| Catalyst evaluation | Hydrolysis reaction rate (µmol•g⁻¹•h⁻¹) | 1438 | 361 | 703 | 918 | 1172 |
| | Esterification reaction rate (mmol·g⁻¹·min⁻¹) | 3.7 | 2.1 | 2.5 | 2.4 | 3.0 |

**[Table 5]**

| | Sample No. | Ex. 21 | Ex. 22 | Ex. 23 | Ex. 24 | Ex. 25 |
|---|---|---|---|---|---|---|
| Raw materials | Wood-based raw material | | | | | |
| | Phenol resin (resole-type) | | | | | |
| | Phenol resin (novolac-type) | | | | | |
| | Furan resin | ○ | ○ | ○ | ○ | ○ |
| | Powder or granules: particle diameter (mm) | 0.45 | 0.45 | 0.45 | 0.45 | 0.45 |
| | Fibers: fiber diameter (µm) | | | | | |
| Carbonization conditions | Carbonization temperature (°C) | Uncarbonized | Uncarbonized | Uncarbonized | Uncarbonized | Uncarbonized |
| Sulfonating agent | Type | Fuming sulfuric acid | Fuming sulfuric acid | Fuming sulfuric acid | Sulfuric acid | Sulfuric acid |
| | Concentration (%) | 11.3 | 11.3 | 11.3 | - | - |
| Sulfonation conditions | Sulfonation temperature (°C) | 100 | 120 | 160 | 25 | 80 |
| | Yield (%) | 132 | 117 | 80 | 120 | 121 |
| | Sulfur content (wt%) | 7.3 | 5.3 | 2.9 | 7.0 | 7.2 |
| | Sulfo groups (mmol/g) | 2.3 | 1.6 | 1.0 | 2.2 | 2.2 |
| Catalyst evaluation | Hydrolysis reaction rate (µmol•g⁻¹•h⁻¹) | 956 | 761 | 390 | 753 | 709 |
| | Esterification reaction rate (mmol·g⁻¹·min⁻¹) | 2.6 | 2.3 | 1.4 | 1.1 | 1.0 |

**[Table 6]**

| | Sample No. | Ex. 26 | Comp. Ex. 1 | Comp. Ex. 2 | Comp. Ex. 3 | Comp. Ex. 4 |
|---|---|---|---|---|---|---|
| Raw materials | Wood-based raw material | | ○ | ○ | ○ | ○ |
| | Phenol resin (resole-type) | ○ | | | | |
| | Phenol resin (novolac-type) | | | | | |
| | Furan resin | | | | | |
| | Powder or granules: particle diameter (mm) | 0.2 | | | | |
| | Fibers: fiber diameter (µm) | | | | | |
| Carbonization conditions | Carbonization temperature (°C) | Uncarbonized | Uncarbonized | 300 | 350 | 400 |
| Sulfonating agent | Type | Fuming sulfuric acid | Fuming sulfuric acid | Fuming sulfuric acid | Fuming sulfuric acid | Fuming sulfuric acid |
| | Concentration (%) | 11.3 | 11.3 | 11.3 | 11.3 | 11.3 |
| Sulfonation conditions | Sulfonation temperature (°C) | 80 | 80 | 80 | 80 | 80 |
| | Yield (%) | 104 | - | - | 56 | 35 |
| | Sulfur content (wt%) | 14.9 | - | - | 7.7 | 2.4 |
| | Sulfo groups (mmol/g) | 4.7 | - | - | 2.4 | 0.7 |
| Catalyst evaluation | Hydrolysis reaction rate (µmol•g⁻¹•h⁻¹) | 1379 | - | - | 923 | 370 |
| | Esterification reaction rate (mmol·g⁻¹·min⁻¹) | 2.6 | - | - | 3.8 | 1.9 |

**[Table 7]**

| | Sample No. | Comp. Ex. 5 | Comp. Ex. 6 | Comp. Ex. 7 |
|---|---|---|---|---|
| Raw materials | Wood-based raw material | | | |
| | Phenol resin (resole-type) | ○ | ○ | ○ |
| | Phenol resin (novolac-type) | | | |
| | Furan resin | | | |
| | Powder or granules: particle diameter (mm) | 0.2 | 0.2 | 0.2 |
| | Fibers: fiber diameter (µm) | | | |
| Carbonization conditions | Carbonization temperature (°C) | 300 | 350 | 400 |
| Sulfonating agent | Type | Fuming sulfuric acid | Fuming sulfuric acid | Fuming sulfuric acid |
| | Concentration (%) | 11.3 | 11.3 | 11.3 |
| Sulfonation conditions | Sulfonation temperature (°C) | 80 | 80 | 80 |
| | Yield (%) | 85 | 87 | 64 |
| | Sulfur content (wt%) | 0.5 | 0.7 | 0.7 |
| | Sulfo groups (mmol/g) | 0.2 | 0.2 | 0.2 |
| Catalyst evaluation | Hydrolysis reaction rate (µmol•g⁻¹•h⁻¹) | 26 | 24 | 44 |
| | Esterification reaction rate (mmol·g⁻¹·min⁻¹) | 0.2 | 0.3 | 0.2 |

**[Table 8]**

| | Sample No. | Comp. Ex. 8 | Comp. Ex. 9 | Comp. Ex. 10 | Comp. Ex. 11 |
|---|---|---|---|---|---|
| Raw materials | Wood-based raw material | | | | |
| | Phenol resin (resole-type) | ○ | ○ | | |
| | Phenol resin (novolac-type) | | | ○ | |
| | Furan resin | | | | ○ |
| | Powder or granules: particle diameter (mm) | 0.2 | 0.2 | | 0.45 |
| | Fibers: fiber diameter (µm) | | | 14 | |
| Carbonization conditions | Carbonization temperature (°C) | 500 | 600 | 350 | 350 |
| Sulfonating agent | Type | Fuming sulfuric acid | Fuming sulfuric acid | Fuming sulfuric acid | Fuming sulfuric acid |
| | Concentration (%) | 11.3 | 11.3 | 11.3 | 11.3 |
| Sulfonation conditions | Sulfonation temperature (°C) | 80 | 80 | 80 | 80 |
| | Yield (%) | 59 | 61 | 99 | 81 |
| | Sulfur content (wt%) | 0.3 | 0.8 | 0.3 | 0.9 |
| | Sulfo groups (mmol/g) | 0.1 | 0.3 | 0.1 | 0.3 |
| Catalyst evaluation | Hydrolysis reaction rate (µmol•g⁻¹•h⁻¹) | 28 | 16 | 25 | 114 |
| | Esterification reaction rate (mmol·g⁻¹·min⁻¹) | 0.1 | 0.2 | 0.3 | 0.3 |

### (Results and Discussion)

All of the resin solid catalysts of the examples demonstrated favorable catalytic action. In addition, based on a comparison with Comparative Examples 5 to 11, the examples, in which the synthetic resin serving as the starting raw material was intentionally not carbonized, demonstrated an increase in the amount of sulfo groups. As a result, the catalytic reactions were presumed to have been activated since the number of active centers of the catalytic reactions per unit weight increased. In the case of carbonizing synthetic resins, various types of functional groups on the surface end up being lost due to calcination. This being the case, since this results in a relative decrease in the number of sites responsible for bonding with sulfo groups, the number of functional groups that bond with sulfo groups can be said to decrease overall. In this manner, the remarkable finding was obtained that further improvement of performance can be realized by omitting customary carbonization treatment.

In addition, carbonization of wood-based raw materials is essential (Comparative Examples 2 to 4) since the starting raw material is unable to withstand the strong acid used during sulfonation in the state of an uncarbonized wood-based raw material (Comparative Example 1). This carbonization results in an unavoidable increase in equipment costs for fuel and the like. In addition, the stability of wood-based raw materials used as starting raw materials can also not be said to be as high as synthetic resins since they are natural materials. Thus, it is highly advantageous to select the synthetic resins of the examples as starting raw materials.

Continuing, a discussion is provided of individual examples. The sulfonating agents used in the examples consisted of fuming sulfuric acid, concentrated sulfuric acid and chlorosulfonic acid, and sulfonation of uncarbonized synthetic resins was possible regardless of the type of sulfonating agent. Next, with respect to the sulfonation temperature, based on a comparison of Example 2 at a nearly normal temperature (room temperature) and Examples 1 and 3, sulfonation is accelerated as the temperature rises and the amount of sulfo groups increases. Although the reaction is able to proceed at room temperature, from the viewpoint of greater efficiency, it is preferable to apply heat in the production stage. Furthermore, decomposition occurred when the uncarbonized synthetic resin was heated to the temperature of Example 5 due to the effects of the heat resistance of the synthetic resin and the potent oxidizing action of the sulfonating agent. Thus, the upper limit of the sulfonation temperature in the case of taking into consideration the temperature range during sulfonation is 200°C or lower, preferably 160°C or lower and more preferably 120°C or lower. The lower limit, although there are no particular limitations thereon, is 40°C or higher and preferably 80°C or higher.

Yield generally exceeded 100% as a result of eliminating combustion loss due to eliminating intermediate carbonization. The increase corresponds to the weight of the sulfo groups bound to the synthetic resin. Furthermore, decomposition occurred during the sulfonation stage in Example 5. This being the case, a yield of 80% or more can be said to be suitable. When considering the amount of sulfo groups, since the amount exceeded 1 mmol/g in all of the examples, this value was taken to be the lower limit. The lower limit is more preferably 2 mmol/g or more.

With respect to the raw material resins, sulfonation was possible in the uncarbonized state in the case of using phenol resin or furan resin as was previously disclosed. Although details as to why these types of resins are effective are unclear, the resistance of these resins to strongly acidic sulfonating agents is thought to have an effect. In addition, sulfonation was possible with phenol resin for both the resole type and novolac type. Moreover, sulfonation was also possible regardless of the form of the resin, whether it be in the form of a powder, granules or fibers. In addition, a sulfo group-modified resin was able to be obtained by re-introducing sulfo groups even in the case of a resin from which the sulfo groups had been eliminated, and was confirmed to be able to be recovered and reused.

### INDUSTRIAL APPLICABILITY

Since the resin solid acid and production method thereof of the present invention enable a starting raw material in the form of a synthetic resin to be processed into a solid acid by sulfonating as is without subjecting to carbonization, production costs can be held to a low level. Moreover, performance aspects of catalytic activity are superior to existing carbonized sulfonation products, making this technology extremely promising as an alternative to the conventional use of sulfuric acid and existing solid acids.

## Claims

1. A resin solid acid that is a sulfo group-modified resin, which uses for a raw material resin thereof a resin selected from among any of uncarbonized phenol resin, uncarbonized furan resin, uncarbonized urea resin, uncarbonized melamine resin and uncarbonized epoxy resin, and is obtained by introducing sulfo groups into the raw material resin.

2. The resin solid acid according to claim 1, wherein the yield of the sulfo group-modified resin based on the weight of the raw material resin is 80% or more.

3. The resin solid acid according to claim 1, wherein the amount of sulfo groups in the sulfo group-modified resin is 1 mmol/g or more.

4. The resin solid acid according to claim 1, wherein the raw material resin is an uncarbonized phenol resin or uncarbonized furan resin.

5. The resin solid acid according to claim 1, wherein the raw material resin is in the form of a powder, granules or fibers.

6. The resin solid acid according to claim 1, wherein the sulfo group-modified resin is used in a catalytic reaction.

7. The resin solid acid according to claim 1, wherein the sulfo group-modified resin is used in a hydrolysis reaction.

8. The resin solid acid according to claim 1, wherein the sulfo group-modified resin is used in an esterification reaction.

9. A method for producing the resin solid acid according to claim 1, in which a sulfo group-modified resin is obtained by comprising:
a step for using as a raw material resin a resin selected from any of uncarbonized phenol resin, uncarbonized furan resin, uncarbonized urea resin, uncarbonized melamine resin and uncarbonized epoxy resin, and adding a sulfonating agent to the raw material resin, and
a step for the heating thereof.

10. The method for producing the resin solid acid according to claim 9, wherein the raw material resin is an uncarbonized phenol resin or uncarbonized furan resin.

11. The method for producing the resin solid acid according to claim 9, wherein the heating temperature in the heating step is 200°C or lower.

12. The method for producing the resin solid acid according to claim 9, wherein the sulfonating agent is sulfuric acid, fuming sulfuric acid or chlorosulfonic acid.
